# EUROPEAN PATENT APPLICATION

(11) **EP 4 427 786 A1**
(43) Date of publication of application: **11.09.2024**
(21) Application number: 22898971.1
(22) Date of filing: 21.11.2022
(51) Int. Cl.: A61M 25/01, A61M 25/00, A61B 17/3207, A61B 17/22, A61B 17/00

(54) **CATHETER SYSTEM**

(30) Priority: 25.11.2021 KR 20210164662
(71) Applicant: IUCF-HYU (INDUSTRY-UNIVERSITY COOPERATION FOUNDATION HANYANG UNIVERSITY), Seoul 04763 (KR)
(72) Inventor: JANG, Gun Hee, Seoul 06326 (KR); PARK, Ji Min, Seoul 05208 (KR)
(74) Representative: Prüfer & Partner mbB Patentanwälte · Rechtsanwälte
(86) International application number: PCT/KR2022/018439
(87) International publication number: WO 2023/096289

(57) **Abstract**

Disclosed is a catheter system. The catheter system includes: a catheter module; and a magnetic robot coupled to catheter module, wherein the magnetic robot idles when an external rotating magnetic field is applied at a coupling position with the catheter module.

## Description

### [Technical Field]

The present invention relates to a catheter system, and more particularly, to a catheter system capable of separating and coupling a magnetic robot by using a catheter module.

### [Background Art]

In general, in order to treat a vascular disease in a blocked or narrowed portion stenosed due to a thrombus or the like, coronary angioplasty is performed in a sequence of inserting a catheter through the femoral artery, widening a blood vessel by a doctor's manual operation and then mounting a device capable of maintaining the widened blood vessel. However, due to the structural characteristics of the catheter, it is difficult to apply the catheter to a complicated blood vessel, and the success of the procedure depends greatly on the skill of the doctor.

In the existing catheter system, a mechanism has been developed to couple and separate a magnetic robot to/from a catheter tube by using a magnetic force of a permanent magnet and a screw coupling.

Korean Registered Patent No. 10-1818400 discloses a mechanism for coupling and separating a magnetic robot to/from a catheter tube by using magnetic force of a permanent magnet. In the case of coupling by the magnetic force of the permanent magnet, a force may be generated in an undesired direction, such as a repulsive force, depending on an alignment direction of the permanent magnet, and a volume of the magnet may limit a function of a catheter tube, such as injection of a contrast medium, injection of drugs, and suction.

Korean Registered Patent No. 10-1749586 discloses a mechanism for coupling and separating a magnetic robot to/from a catheter tube by using a screw coupling. In the case of screw coupling, the coupling may fail when the magnetic robot and the catheter tube are not accurately centered.

In addition, in the above two coupling schemes, it is difficult to steer the catheter tube according to the alignment direction between the permanent magnet of the magnetic robot and an external magnetic field while the magnetic robot is coupled to the catheter tube. Specifically, when the external magnetic field is applied while the directions of the permanent magnet of the magnetic robot and the external magnetic field are not aligned, the magnetic robot may be separated in a process in which the magnetic robot is rotated and aligned in the direction of the external magnetic field in the case of magnetic force coupling, and the catheter tube may be twisted due to the rotation of the magnetic robot in the case of screw coupling.

### [Disclosure]

### [Technical Problem]

The present invention provides a catheter system capable of accurately steering a catheter module in the direction of an external magnetic field.

In addition, the present invention provides a catheter system capable of injecting drugs while a catheter module and the magnetic robot are coupled.

In addition, the present invention provides a catheter system capable of accurately centering a magnetic robot to a catheter module.

### [Technical Solution]

The catheter system according to the present invention includes: a catheter module; and a magnetic robot to be coupled with the catheter module, wherein the magnetic robot may idle when an external rotating magnetic field is applied at a coupling position with the catheter module.

In addition, the catheter module may include: a coupling ring sequentially formed therein with a coupling space and a decoupling space having an inner diameter larger than an inner diameter of the coupling space; and a catheter formed of a flexible material and coupled to the coupling ring. Meanwhile, the magnetic robot may include a body having a screw thread shape and formed at a rear end thereof with a coupling region inserted into the coupling ring, and a drive magnet provided inside the body. The coupling region of the magnetic robot may include a first region having an outer diameter corresponding to the inner diameter of the coupling space; and a second region positioned between the first region and the drive magnet and having an outer diameter smaller than the outer diameter of the first region.

In addition, the coupling space may be formed on an inner circumferential surface thereof with a spiral groove, and the first region may be formed on an outer circumferential surface thereof with a spiral protrusion fastened to the spiral groove.

In addition, the first region may have a length equal to or less than a length of the decoupling space, and the second region may have a length equal to or greater than a length of the coupling space.

In addition, the first region may have a flow path recessed inward from the outer circumferential surface of the first region in a longitudinal direction of the body.

In addition, the body may be formed at a front end thereof with a drilling tip, and the body may be formed on an outer circumferential surface thereof with a spiral protrusion.

### [Advantageous Effects]

According to the present invention, since the magnetic robot may be co-rotated while the catheter module is coupled to the magnetic robot, the magnetic robot can be aligned in the direction of the external magnetic field without twisting the catheter.

In addition, according to the present invention, a drug can be injected into the blood vessel through a gap between the coupling ring and the coupling region while the catheter module is coupled to the magnetic robot.

In addition, according to the present invention, the magnetic robot can be accurately centered to the catheter module under control of the external magnetic field and suction of the catheter module.

### [Description of Drawings]

FIG. 1 is a perspective view showing a catheter system according to an embodiment of the present invention##.
FIG. 2 is a perspective view showing a state in which a catheter module and a magnetic robot are separated from each other in the catheter system of FIG. 1.
FIG. 3 is a sectional view showing the catheter system of FIG. 1.
FIGS. 4 and 5 are views showing a magnetic robot according to another embodiment of the present invention.
FIGS. 6 to 10 are views sequentially showing a process of treating a vascular disease of a human body by using the catheter system of the present invention.
FIG. 11 shows states in which the catheter module according to the embodiment of the present invention is steered by controlling an external magnetic field.
FIG. 12 is a view showing a state in which a catheter module according to a comparative example is steered by controlling an external magnetic field.

### [Best Mode]

The catheter system according to an embodiments of the present invention includes: a catheter module; and a magnetic robot to be coupled with the catheter module, wherein the magnetic robot may idle when an external rotating magnetic field is applied at a coupling position with the catheter module.

### [Mode for Invention]

Hereinafter, exemplary embodiments of the present invention will be described in detail with reference to the accompanying drawings. However, the technical idea of the present invention is not limited to the exemplary embodiments described herein and may be embodied in other forms. Further, the embodiments are provided to enable contents disclosed herein to be thorough and complete and provided to enable those skilled in the art to fully understand the idea of the present invention.

In the specification herein, when one component is mentioned as being on other component, it signifies that the one component may be placed directly on the other component or a third component may be interposed therebetween. In addition, in the drawings, thicknesses of films and areas may be exaggerated to effectively describe the technology of the present invention.

In addition, although terms such as first, second and third are used to describe various components in various embodiments of the present specification, the components will not be limited by the terms. The above terms are used merely to distinguish one component from another. Accordingly, a first component referred to in one component may be referred to as a second component in another embodiment. Each embodiment described and illustrated herein may also include a complementary embodiment. In addition, the term "and/or" is used herein to include at least one of the components listed before and after the term.

The singular expression herein includes a plural expression unless the context clearly specifies otherwise. In addition, it will be understood that the term such as "include" or "have" herein is intended to designate the presence of feature, number, step, component, or a combination thereof recited in the specification, and does not preclude the possibility of the presence or addition of one or more other features, numbers, steps, components, or combinations thereof. In addition, the term "connection" is used herein to include both indirectly connecting a plurality of components and directly connecting the components.

In addition, in the following description of the embodiments of the present invention, the detailed description of known functions and configurations incorporated herein will be omitted when it possibly makes the subject matter of the present invention unclear unnecessarily.

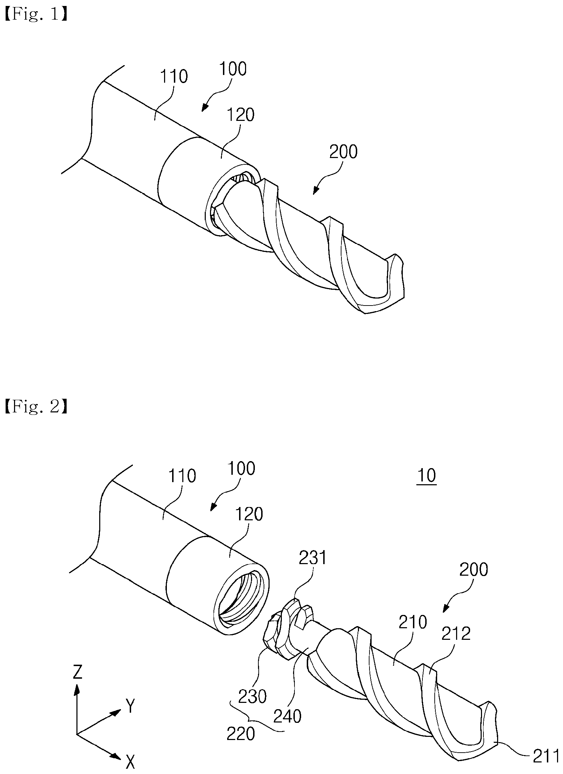

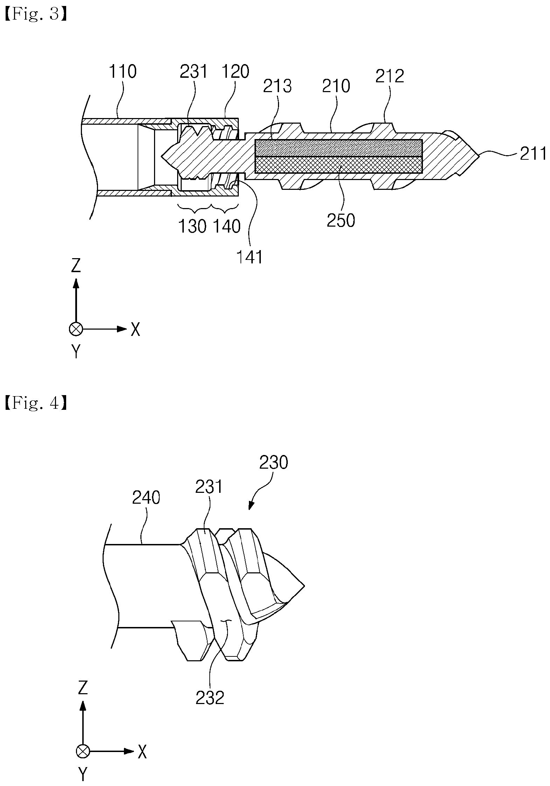

FIG. 1 is a perspective view illustrating a catheter system according to an embodiment of the present invention; FIG. 2 is a perspective view illustrating a state in which a catheter module and a magnetic robot are separated from each other in the catheter system of FIG. 1; and FIG. 3 is a sectional view illustrating the catheter system of FIG. 1.

Referring to FIGS. 1 to 3, a catheter system 10 includes a catheter module 100, a magnetic robot 200, and a magnetic field generator (not shown). The catheter module 100 may be coupled to and separated from the magnetic robot 200, and may be deployed the magnetic robot 200 to the vicinity of the lesion while the magnetic robot 200 is coupled.

The catheter module 100 includes a catheter 110 and a coupling ring 120.

The catheter 110 has a tube shape having a predetermined length and may be inserted into a body tubular tissue. The catheter 110 is formed of a deformable flexible material.

The coupling ring 120 is fixedly coupled to a front end of the catheter 110. The coupling ring 120 has a predetermined length and has an outer diameter corresponding to the catheter 110. A decoupling space 130 and a coupling space 140 are sequentially formed inside the coupling ring 120. The decoupling space 130 is formed at a rear end of the coupling ring 120 adjacent to the catheter 110, and the coupling space 140 is formed adjacent to a front end of the coupling ring 120. An inner diameter of the decoupling space 130 and an inner diameter of the coupling space 140 may be different from each other. According to the embodiment, the inner diameter of the decoupling space 130 may be greater than the inner diameter of the coupling space 140.

A spiral groove 141 is formed along a circumference of the inner diameter of the coupling space 140. The spiral groove 141 extends from the front end of the coupling ring 120 to just before the decoupling space 130.

The magnetic robot 200 includes a body 210 and a drive magnet 250.

The body 210 has a predetermined length and is formed of a non-magnetic material. A drilling tip 211 is formed at a front end of the body 210, and a spiral protrusion 212 is formed on an outer circumferential surface of the body. An inner space 213 is formed inside the body 210.

A coupling region 220 is provided at a rear end of the body 210. The coupling region 220 refers to a region inserted into the catheter module 100 when the magnetic robot 200 is coupled to the catheter module 100, and is provided with a length corresponding to or longer than the coupling ring 120.

The coupling region 220 includes a first region 230 and a second region 240. The first region 230 is positioned at the rear end of the body 210, and the second region 240 is positioned between the first region 210 and the inner space 213.

The first region 230 has an outer diameter corresponding to an inner diameter of the coupling space 140, and the second region 240 has an outer diameter smaller than the first region 230. A spiral protrusion 231 may be formed along an outer circumferential surface of the first region 230. The spiral protrusion 231 may be fastened to the spiral groove 141 formed in the coupling ring 120. A length of the first region 230 in the longitudinal direction of the body 210 may be equal to or less than a length of the decoupling space 130.

The second region 240 has the outer diameter smaller than the outer diameter of the first region 230 and the inner diameter of the coupling space 140. A length of the second region 240 in the longitudinal direction of the body 210 may be equal to or greater than a length of the coupling space 140.

Since the outer diameter of the first region 230 is smaller than the inner diameter of the decoupling space 130 and the outer diameter of the second region 240 is smaller than the inner diameter of the coupling space 140 while the magnetic robot 200 is coupled to the catheter module 100, a gap is present between the coupling ring 120 and the coupling region 220. The catheter module 100 may supply the drug into the blood vessel through the gap while the magnetic robot 200 is coupled thereto.

The drive magnet 250 is inserted into the inner space 213 of the body 210 and fixedly coupled to the body 210. The drive magnet 250 is integrally driven with the body 210. The drive magnet 250 has a cylindrical shape and is magnetized in a radial direction of the body 210.

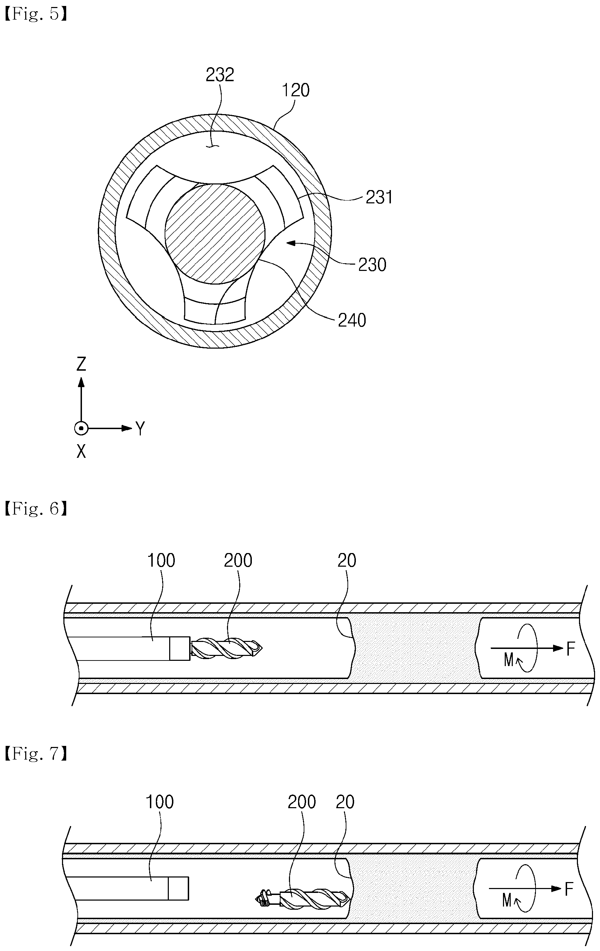

FIGS. 4 and 5 are views showing a magnetic robot according to another embodiment of the present invention.

Referring to FIGS. 4 and 5, A flow path 232 may be formed in the first region 230 of the coupling region 220. The flow path 232 may be recessed inward from the outer circumferential surface of the first region 230 by a predetermined depth. A plurality of flow paths 232 may be spaced apart from each other along the circumference of the first region 230, and formed in the longitudinal direction of the body 210. Since the gap between the coupling ring 120 and the first region 230 is increased by the flow path 232 when the magnetic robot 200 is coupled to the catheter module 100, the drug may be smoothly supplied through the flow path 232.

Hereinafter, a process of treating a vascular disease of a human body using the above-described catheter system 10 will be described in detail.

FIGS. 6 to 10 are views sequentially showing a process of treating a vascular disease of a human body by using the catheter system of the present invention.

First, referring to FIG. 6, the magnetic robot 200 is moved to the vicinity of a lesion 20 while being coupled to the catheter module 100. According to one embodiment, the magnetic robot 200 may inject drugs and contrast agents while being coupled to the catheter module 100. The drugs and the contrast agents may be supplied through the catheter module 100 and supplied toward the lesion 20 through the gap between the coupling ring 120 and the coupling region 220. When a rotating magnetic field M is applied from the magnetic field generator, the magnetic robot 200 is rotated and a propulsive force F is generated by the spiral protrusion 212 formed on the outer circumferential surface of the magnetic robot. The magnetic robot 200 is moved forward by the propulsive force F, and the coupling region 220 is separated from the catheter module 100 when passing through the coupling space 140.

Referring to FIG. 7, the magnetic robot 200 separated from the catheter module 100 generates the propulsive force F while being rotated by the rotating magnetic field M and moves near the lesion 20. The magnetic robot 200 having arrived at the lesion 20 is rotated at a high speed to remove the lesion 20. At this timing, the drugs and the contrast agents may be injected through the catheter module 100.

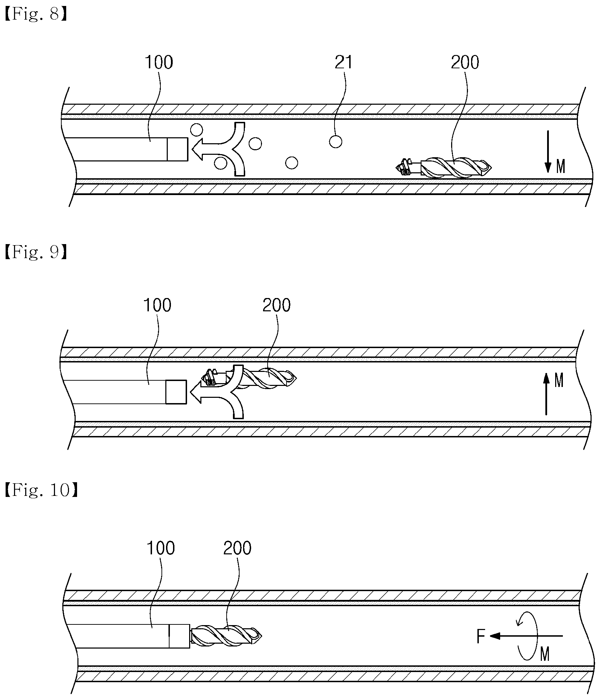

Referring to FIG. 8, fragments 21 generated during drilling operation of the magnetic robot 200 may be sucked into the catheter module 100 by suction in the catheter module 100. At this timing, the propulsive force may be generated by controlling the external magnetic field or the magnetic robot 200 may be fixed to a blood vessel wall in order to prevent the magnetic robot from being sucked into the catheter module 100 side. Accordingly, the catheter module 100 may suck fragments of the lesion and blood only.

Referring to FIG. 9, when the suction of the fragments 21 is completed, the rotating magnetic field M is applied to rotate and float the magnetic robot 200, and the magnetic robot 200 is aligned with the catheter module 100. In this state, when a negative pressure is applied into the catheter module 100, the blood is introduced into the catheter module 100, and the magnetic robot 200 is moved toward the catheter module 100 due to the flow of the blood. The magnetic robot 200 may sink due to the self-load during the backward movement of the magnetic robot 200, and accordingly the alignment with the catheter module 100 may be disturbed. In order to prevent this, the external magnetic field M may be generated toward the reverse direction of gravity, that is, upward, thereby offsetting the influence of the self-load.

Referring to FIG. 10, when the coupling region 220 of the magnetic robot 200 is positioned inside the coupling ring 120, a rotating magnetic field M is generated in the reverse direction. The magnetic robot 200 is rotated in the reverse direction by the rotating magnetic field M to generate the propulsive force F rearward, and the spiral protrusion 231 formed in the first region 230 of the coupling region 220 is coupled to the spiral groove 141 formed inside the coupling space 140. When the magnetic robot 200 is additionally rotated, the first region 230 is unfastened with the coupling space 140, and the first region 230 is positioned in the decoupling space 130.

As described above, in the catheter system 10 according to the present invention, the magnetic robot 200 may idle while the magnetic robot 200 and the catheter module 100 are coupled to each other.

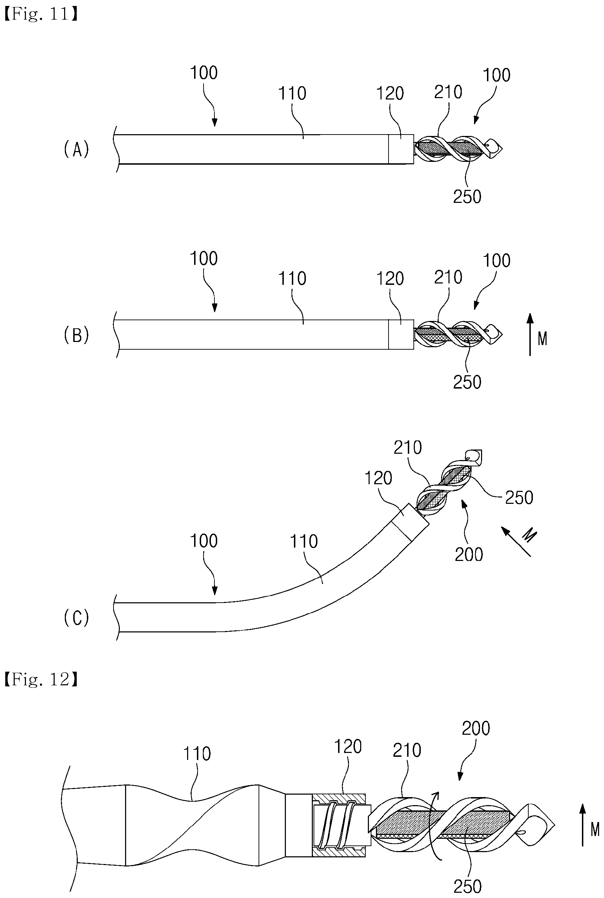

FIG. 11 shows states in which the catheter module according to the embodiment of the present invention is steered by controlling an external magnetic field. FIG. 12 is a view showing a state in which a catheter module according to a comparative example is steered by controlling an external magnetic field. In the comparative example, the magnetic robot 200 is steered under control of the external magnetic field while being screwed and fixedly coupled to the catheter module 100. In FIGS. 11 and 12, the drive magnets are expressed to be exposed to the outside to show that the drive magnets are aligned in the direction of the external magnetic field.

First, referring to FIG. 11, In the process of deploying the magnetic robot 200 in the vicinity of the lesion by the catheter module 100, the catheter module 100 is steered while the drive magnet 250 is aligned in the direction of the external magnetic field M.

As shown in FIG. 12, when the external magnetic field M is applied in a state in which the magnetic robot 200 is fixedly coupled to the catheter module 100 and the direction of the magnetic field of the drive magnet 250 is not aligned with the direction of the external magnetic field, the drive magnet 250 is rotated and aligned in the direction of the external magnetic field M. At this time, when the drive magnet 250 is rotated in the coupling direction between the magnetic robot 200 and the catheter module 100, the catheter module 100 is rotated together, thereby twisting the catheter 110. On the other hand, when the drive magnet 250 is rotated in the decoupling direction between the magnetic robot 200 and the catheter module 100, the coupling between the magnetic robot 200 and the catheter module 100 may be decoupled. In addition, when the drive magnet 250 has a large rotation size, a coupling portion between the magnetic robot 200 and the catheter module 100 may be damaged. In this state, when the catheter module 100 is steered, it is difficult to accurately control the direction.

However, in the present invention, when the external magnetic field M is applied while the direction of the magnetic field of the drive magnet 250 is not aligned with the direction of the external magnetic field, the magnetic robot 200 idles so that the direction of the magnetic field of the drive magnet 250 is aligned in the direction of the external magnetic field M. Thus, the magnetic robot 200 can be aligned without twisting the catheter 110.

Although the present invention has been described in detail using exemplary embodiments, the scope of the present invention is not limited to a specific embodiment and will be interpreted by the appended claims. Further, it will be understood by a person having ordinary skill in the art that many modifications and variations are possible without departing from the scope of the present invention.

### [Industrial Applicability]

The catheter system according to an embodiment of the present invention may be used for the treatment of vascular diseases.

## Claims

1. A catheter system comprising:
a catheter module; and
a magnetic robot coupled to catheter module, wherein
the magnetic robot idles when an external rotating magnetic field is applied at a coupling position with the catheter module.

2. The catheter system of claim 1, wherein the catheter module includes:
a coupling ring sequentially formed therein with a coupling space and a decoupling space having an inner diameter larger than an inner diameter of the coupling space; and
a catheter formed of a flexible material and coupled to the coupling ring, wherein
the magnetic robot includes:
a body formed at a rear end thereof with a coupling region inserted into the coupling ring; and
a drive magnet provided inside the body, and wherein the coupling region has:
a first region having an outer diameter corresponding to the inner diameter of the coupling space; and
a second region positioned between the first region and the drive magnet and having an outer diameter smaller than the outer diameter of the first region.

3. The catheter system of claim 2, wherein the coupling space is formed on an inner circumferential surface thereof with a spiral groove, and
the first region is formed on an outer circumferential surface thereof with a spiral protrusion fastened to the spiral groove.

4. The catheter system of claim 2, wherein the first region has a length equal to or less than a length of the decoupling space, and
the second region has a length equal to or greater than a length of the coupling space.

5. The catheter system of claim 2, wherein the first region has a flow path recessed inward from an outer circumferential surface of the first region in a longitudinal direction of the body.

6. The catheter system of claim 2, wherein the body is formed at a front end thereof with a drilling tip, and formed on an outer circumferential surface thereof with a spiral protrusion.
